# EUROPÄISCHE PATENTSCHRIFT

(11) **EP 4 370 061 B1**
(45) Veröffentlichungstag und Bekanntmachung des Hinweises auf die Patenterteilung: **09.04.2025**
(21) Anmeldenummer: 22746982.2
(22) Anmeldetag: 06.07.2022
(51) Int. Cl.: A61F 2/16, G02B 27/01, G02F 1/133, G03H 1/22

(54) **KÜNSTLICHE AUGENLINSE MIT INTEGRIERTER BILDPROJEKTIONSVORRICHTUNG, ELEKTRONISCHES INFORMATIONSSYSTEM**
ARTIFICIAL EYE LENS WITH INTEGRATED IMAGE PROJECTION DEVICE, ELECTRONIC INFORMATION SYSTEM
CRISTALLIN OCULAIRE ARTIFICIEL AVEC DISPOSITIF DE PROJECTION D'IMAGE INTÉGRÉ, SYSTÈME D'INFORMATION ÉLECTRONIQUE

(30) Priorität: 13.07.2021 DE 102021118003
(43) Veröffentlichungstag der Anmeldung: 22.05.2024
(73) Patentinhaber: Carl Zeiss Meditec AG, 07745 Jena (DE)
(72) Erfinder: SCHARSICH, Christina, 14471 Potsdam (DE); GERLACH, Mario, 16548 Glienicke-Nordbahn (DE)
(74) Vertreter: Hofstetter, Schurack & Partner
(86) Internationale Anmeldenummer: PCT/EP2022/068695
(87) Internationale Veröffentlichungsnummer: WO 2023/285228

(56) Entgegenhaltungen:
- US-A1- 2019 150 731
- US-A1- 2019 274 822
- US-A1- 2021 015 604

## Beschreibung

### Technisches Gebiet

Ein Aspekt der Erfindung betrifft eine künstliche Augenlinse mit zumindest einem optischen Teil. Der optische Teil weist eine Vorderseite auf. Diese Vorderseite ist bestimmungsgemäß örtlich so zu verstehen, dass sie dann, wenn die künstliche Augenlinse am oder im Auge angeordnet ist, der Netzhaut, insbesondere dem Sehnerv des Auges abgewandt ist. Diese Vorderseite ist also der Umgebung der Person zugewandt. Der optische Teil weist eine der Vorderseite gegenüberliegende Rückseite auf. Diese ist bestimmungsgemäß so zu verstehen, dass sie dann, wenn die künstliche Augenlinse am oder im Auge angeordnet ist, der Netzhaut, insbesondere dem Sehnerv, des Auges zugewandt ist. Diese Rückseite ist daher der vorderen Umgebung der Person beziehungsweise der vorderen Umgebung des Auges abgewandt.

### Stand der Technik

Künstliche Augenlinsen sind beispielsweise als Intraokularlinsen bekannt. Eine Intraokularlinse weist einen optischen Teil auf, an den randseitig zumindest eine Haptik anschließen kann. Mittels dieser Haptik kann die Intraokularlinse im Auge, beispielsweise in einem Kapselsack, gehalten werden. Die Haptik ist dabei Bestandteil der Intraokularlinse und weist keine optische Abbildungseigenschaft auf, wie dies jedoch der optische Teil hat.

Ein optischer Teil einer künstlichen Augenlinse ist üblicherweise aus einem Kunststoffmaterial, wie beispielsweise einem Polymermaterial, aufgebaut. Dieser optische Teil weist durch seine Formgebung eine spezifische optische Abbildungseigenschaft auf. Diese optische Abbildungseigenschaft kann refraktiv und/oder diffraktiv sein. Üblicherweise sind in dem Zusammenhang spezifische optische Strukturen in dem optischen Teil ausgebildet oder angeordnet. Damit kann das auf die künstliche Augenlinse aus der Umgebung einfallende Licht durch die optische Abbildungseigenschaft des optischen Teils spezifisch gelenkt werden.

Darüber hinaus ist es auch bekannt, dass eine derartige künstliche Augenlinse im optischen Teil eine Apertur beziehungsweise eine Lochblende aufweisen kann. Damit kann die Schärfentiefe der künstlichen Augenlinse individuell gestaltet werden beziehungsweise verbessert werden.

Eine derartige künstliche Augenlinse ist beispielsweise aus der DE 10 2018 118 714 A1 bekannt.

Darüber hinaus sind auch künstliche Augenlinsen bekannt, die zusätzlich zur optischen Funktionalität des optischen Teils weitere integrierte Funktionalitäten aufweisen. In dem Zusammenhang ist beispielsweise aus der US 2008/0208335 A1 bekannt, dass eine künstliche Augenlinse auch ein elektroaktives Element aufweisen kann. Dieses kann beispielsweise auch eine Solarzelle sein. Das elektroaktive Element kann auch seinen Brechungsindex verändern. Dieses elektroaktive Element kann innenliegend in einem Linsenkörper der künstlichen Augenlinse angeordnet sein. Dieses elektroaktive Element kann auch aus mehreren Schichten aufgebaut sein. Es sind dort auch verschiedene Ringzonen mit unterschiedlichen Funktionen vorgesehen. Die verschiedenen Ringzonen sind in radialer Richtung zur optischen Hauptachse des dortigen Linsenkörpers radial aneinander anschließend und diesbezüglich radial überlappungsfrei angeordnet.

Aus der US 2019 / 0 274 822 A1 ist eine Intraokularlinse mit einem Display am optischen Teil bekannt. Aus der US 2016 / 0 256 260 A1 ist ein Augenimplantat bekannt, welches eine spezifische Haltestruktur aufweist. Aus der US 2021 / 015 604 A1 ist eine Intraokularlinse mit elektronisch verstellbarer Lochblende bekannt.

### Darstellung der Erfindung

Es ist Aufgabe der vorliegenden Erfindung, eine künstliche Augenlinse und ein elektronisches Informationssystem zu schaffen, welche beziehungsweise welches eine verbesserte und umfänglichere optische Informationsdarstellung ermöglicht.

Diese Aufgabe wird durch eine künstliche Augenlinse und ein elektronisches Informationssystem gemäß den unabhängigen Ansprüchen gelöst.

Ein Aspekt der Erfindung betrifft eine künstliche Augenlinse mit zumindest einem optischen Teil. Der optische Teil weist eine Vorderseite auf. Diese Vorderseite ist bestimmungsgemäß örtlich so zu verstehen, dass sie dann, wenn die künstliche Augenlinse im Auge angeordnet ist, der Netzhaut, insbesondere dem Sehnerv, des Auges abgewandt ist. Diese Vorderseite ist also der vorderen Umgebung der Person zugewandt, wenn die künstliche Augenlinse im Auge angeordnet ist. Der optische Teil weist eine der Vorderseite gegenüberliegende Rückseite auf. Diese ist bestimmungsgemäß so zu verstehen, dass sie dann, wenn die künstliche Augenlinse im Auge angeordnet ist, der Netzhaut, insbesondere dem Sehnerv des Auges zugewandt ist. Diese Rückseite ist daher der vorderen Umgebung der Person beziehungsweise der vorderen Umgebung des Auges abgewandt.

Die künstliche Augenlinse weist eine Bildprojektionsvorrichtung auf. Mit dieser ist eine durch die Bildprojektionsvorrichtung selbst erzeugte optische Information zumindest auf die Rückseite des optischen Teils projizierbar beziehungsweise ist darauf projiziert. Die Bildprojektionsvorrichtung ist also so am oder im optischen Teil angeordnet, dass sie das von ihr emittierte Lichtbild direkt zur Rückseite strahlt. Diese künstliche Augenlinse weist somit durch die Bildprojektionsvorrichtung eine eigenständige Komponente auf, die eine eigene Bilderzeugung ermöglicht. Diese Bildprojektionsvorrichtung ermöglicht daher eine Bilderzeugung unabhängig von der optischen Abbildungseigenschaft des optischen Teils selbst. Mit dem optischen Teil kann Licht aus der Umgebung, das auf die künstliche Augenlinse trifft, aufgrund der Ausgestaltung des optischen Teils und der damit einhergehenden inhärenten optischen Abbildungseigenschaft dieses einfallende Umgebungslicht entsprechend umgelenkt werden. Die Bildprojektionsvorrichtung hingegen arbeitet nicht mit dem auf die Linse einfallenden Umgebungslicht, sondern erzeugt das Bild selbst. Dies erfolgt insbesondere unabhängig von dem auf die künstliche Augenlinse aus der Umgebung einfallenden Umgebungslicht. Die Bildprojektionsvorrichtung ist daher zur Erzeugung von optischen Informationen unabhängig von dem auf die künstliche Augenlinse aus der Umgebung einfallenden Umgebungslicht ausgebildet.

Möglich ist es daher in dem Zusammenhang auch, dass im eingesetzten Zustand dieser künstlichen Augenlinse in das Auge von der die künstliche Augenlinse tragenden Person auch gleichzeitig optische Informationen der Bildprojektionsvorrichtung und die dazu unterschiedlich und separat durch das einfallende Umgebungslicht charakterisierten, weiteren optischen Wahrnehmungsmöglichkeiten wahrgenommen werden können.

Insbesondere kann durch eine derartige Bildprojektionsvorrichtung und deren spezifische Projektionsrichtung, nämlich zumindest direkt auf die Rückseite des optischen Teils, auch erreicht werden, dass im eingesetzten Zustand der künstlichen Augenlinse an das Auge oder in das Auge diese projizierten Informationen auf der Netzhaut des Auges auftreffen und somit durch das natürliche Auge erfasst und wahrgenommen werden können. Durch eine derartige definierte Projektionsrichtung der Bildprojektionsvorrichtung können daher gezielt Informationen zumindest auf diese Rückseite projiziert werden, um diese einfach und eindeutig auf die Netzhaut des Auges projizieren zu können und somit von der Person, die die künstliche Augenlinse im Auge trägt, auch zuverlässig erkannt werden.

Es ist die Bildprojektionsvorrichtung dazu ausgebildet und angeordnet, eine durch die Bildprojektionsvorrichtung selbst erzeugte optische Information durch die Rückseite des optischen Teils hindurch aus dem optischen Teil heraus nach hinten zu projizieren. Damit kann die spezifisch erzeugte optische Information der Bildprojektionsvorrichtung in eine ganz spezifische Position hinter der Rückseite der künstlichen Augenlinse abgestrahlt werden. Dies ist insbesondere dann sehr vorteilhaft, um diese optische Information auch an die Netzhaut des Auges leiten beziehungsweise strahlen zu können. Dadurch kann diese optische Information quasi auch direkt auf diese Netzhaut der Bildprojektionsvorrichtung projiziert werde, wenn die künstliche Augenlinse im Auge angeordnet ist. Die damit erzeugte optische Information kann daher sehr eindeutig und auch scharf von dem Auge erkannt werden.

Damit ist es besonders vorteilhaft und exakt möglich, optische Zusatzinformationen zusätzlich zu den aus der Umgebung wahrgenommenen optischen Informationen auf die Netzhaut zu projizieren, um von der Person wahrgenommen werden zu können. Damit ist es in besonders vorteilhafter Weise auch ermöglicht, derartige künstlich durch die Bildprojektionsvorrichtung erzeugten optischen Informationen auf die Netzhaut zu projizieren. Damit ist es besonders vorteilhaft ermöglicht, dass auch unabhängig von den Umgebungsinformationen, die durch das Auge wahrgenommen werden, anderweitige optische Informationen durch die Bildprojektionsvorrichtung auf die Netzhaut projiziert werden. In einem anderen Ausführungsbeispiel kann auch vorgesehen sein, dass diese optischen Informationen durch die Bildprojektionsvorrichtung Zusatzinformationen zu ganz konkreten Objekten in der Umgebung der Person zugehörig sind. Damit kann eine Person durch diese optischen Informationen in der Bildprojektionsvorrichtung zusätzlich über Objekte, die sich in der Umgebung der Person befinden und die durch die Person durch das Auge, insbesondere auch aktuell, wahrgenommen werden können, informiert werden. Dies können Erläuterungsinformationen oder Hilfsinformationen oder dergleichen sein. Ebenso können dies dann auch Informationen zur Orientierung einer Person sein. Beispielsweise können diese erzeugten optischen Informationen der Bildprojektionsvorrichtung Erläuterungen zu Wegbeschreibungen und/oder zu beispielsweise einem spezifischen Eingang eines Gebäudes und/oder zur Lage von spezifischen Gebäudebereichen in einem Gebäude, wie beispielsweise eine Arztpraxis oder dergleichen, sein. Ebenso ist es jedoch auch möglich, dass aktuelle Positionsinformationen der Person als derartige optische Informationen durch die Bildprojektionsvorrichtung erzeugt werden. Damit kann beispielsweise auch eine aktuelle Position einer Person relativ zu anderen Objekten in der Umgebung angezeigt werden. Besonders vorteilhaft ist es nicht nur in dem Zusammenhang, dass Orientierungs- und Führungsinformationen durch die Bildprojektionsvorrichtung als optische Informationen generiert und zumindest auf die Rückseite der künstlichen Augenlinse, insbesondere darüber hinaus, entsprechend projiziert werden. Damit kann insbesondere gegebenenfalls auch bei einer Orientierungssuche einer Person in der Umgebung unterstützend mitgewirkt werden. Besonders vorteilhaft ist dies dann gegebenenfalls auch für hilfsbedürftige Personen. Beispielsweise dann, wenn nach Erkrankung, wie auch beispielsweise Demenz oder dergleichen, derartige Orientierungs- und Führungsinformationen vorteilhaft sind. Nicht nur zur Wegfindung und Orientierung können derartige Informationen hilfsweise unterstützend beitragen. Ebenso können beispielsweise Informationen zur Medikamenteneinnahme nicht nur für einen derartigen Personenkreis als optische Informationen durch die Bildprojektionsvorrichtung generiert werden. Beispielsweise kann in dem Zusammenhang eine Erinnerungsinformation als optische Information durch die Bildprojektionsvorrichtung erzeugt werden. Dies kann beispielsweise der Zeitpunkt und/oder die Menge und/oder die Art der Einnahme eines Medikaments als optische Information sein. Möglich ist jedoch jegliche andere weitere optische Information, wie beispielsweise eine Terminerinnerung oder dergleichen. Darüber hinaus ist es auch möglich, dass als Hinweisinformation beispielsweise der Eingang einer E-Mail und/oder eines Telefonanrufs, beispielsweise auf einem Mobilfunkgerät, als optische Information durch die Bildprojektionsvorrichtung generiert wird und diesbezüglich in die genannte Projektionsrichtung die optische Information abgestrahlt wird.

Insbesondere können derartige optische Informationen auch für spezifische Personen, insbesondere hilfsbedürftige Personen, Wegweiser in einem eigenen Haus oder in einer eigenen Wohnung sein.

In einem Ausführungsbeispiel können als optische Informationen einfache Symbole und/oder Buchstaben und/oder Zahlen durch die Bildprojektionsvorrichtung erzeugt und entsprechend projiziert werden. Möglich ist es zusätzlich oder anstatt dazu auch, dass Bilder erzeugt werden. Diese können dann als ganz spezifische Szenarien mit mehreren Objekten virtuell und/oder symbolhaft oder auch als sehr realitätsnahe Darstellung erzeugt und projiziert werden.

In dem Zusammenhang ist es in einem Ausführungsbeispiel möglich, dass die Bildprojektionsvorrichtung als Augmented-Reality-(Erweiterte Realität)-Bildprojektionsvorrichtung ausgebildet ist. Damit ist es insbesondere auch möglich, dass abhängig von der Blickrichtung der Person eine kontaktanaloge Darstellung der optischen Informationen, die von der Bildprojektionsvorrichtung erzeugt werden, mit Objekten, die in der Umgebung der Person wahrgenommen werden, ermöglicht ist.

Auch Patienten, die an altersbedingter Makuladegeneration (AMD) erkrankt sind, können von einer Intraokularlinse gemäß dem oben genannten Aspekt oder einem vorteilhaften Ausführungsbeispiel davon profitieren. Denn in einem Ausführungsbeispiel kann die Bildprojektionsvorrichtung dazu ausgebildet sein, dass sie die Größe des projizierten Bildes und/oder den Projektionsort auf die Rückseite, insbesondere hinter die Rückseite, der Intraokularlinse, insbesondere auf die Retina verändern kann. Insbesondere ist dies durch die Art und/oder Größe des Hologramms möglich, das diesbezüglich veränderbar ist. Insbesondere erfolgt dies abhängig von dem Grad der Makuladegeneration. Dadurch ist auch eine dynamische Anpassung der Bildprojektion an den Krankheitsverlauf möglich.

In einem Ausführungsbeispiel ist die Bildprojektionsvorrichtung zur Erzeugung eines 3D-Bilds ausgebildet. Besonders vorteilhaft ist es, wenn die Bildprojektionsvorrichtung zur Erzeugung eines Hologramms ausgebildet ist. Dadurch ist die Vielfältigkeit und Flexibilität der optischen Informationsdarstellung nochmals verbessert. Eine noch realitätsnähere und überwiegend intuitiv noch leichter wahrnehmbare und verständliche Informationsdarstellung durch die Bildprojektionsvorrichtung ist dadurch ermöglicht.

In einem Ausführungsbeispiel weist die Bildprojektionsvorrichtung zumindest eine kohärente Lichtquelle auf. Diese kohärente Lichtquelle kann beispielsweise eine Laserdiode sein. Derartige Lichtquellen sind einerseits sehr kompakt aufgebaut. Andererseits ermöglichen sie die Erzeugung eines Hologramms als optische Information.

In einem Ausführungsbeispiel weist die künstliche Augenlinse zumindest eine Haptik auf. Die Haptik ist mit dem optischen Teil verbunden. Insbesondere ist sie diesbezüglich an einem radial äußeren Rand angeordnet, insbesondere direkt an dem optischen Teil radial anschließend angeordnet. Die Haptik kann als Bügel ausgebildet sein. Dieser Bügel kann nur mit einem Ende an dem optischen Teil direkt angeordnet sein. Das zweite Ende eines derartigen Bügels kann freiliegend beziehungsweise freikragend angeordnet sein. In einem weiteren Ausführungsbeispiel kann die Haptik mit ihren beiden Enden jeweils direkt, insbesondere an unterschiedlichen Stellen, an dem optischen Teil enden. Insbesondere kann sie auch hier wieder an einem radial äußeren Umfangsrand des optischen Teils enden. Eine Haptik ist bestimmungsgemäß dazu vorgesehen, eine künstliche Augenlinse, wenn sie als Intraokularlinse ausgebildet ist, im Auge, beispielsweise in einem Kapselsack, zu halten. Eine Haptik weist üblicherweise keine optische Abbildungseigenschaft auf.

In einem Ausführungsbeispiel ist die kohärente Lichtquelle an der Haptik angeordnet. Damit ist der optische Teil nicht beeinträchtigt. Insbesondere wird dabei der optische Teil diesbezüglich nicht in seiner optischen Abbildungseigenschaft eingeschränkt. Die Haptik dient andererseits durch ein derartiges Ausführungsbeispiel als multifunktionelle Komponente. Denn sie dient dann als Halterung der Intraokularlinse im Auge, andererseits als Träger für diese kohärente Lichtquelle.

Es kann in einem Ausführungsbeispiel vorgesehen sein, dass die kohärente Lichtquelle an einer Oberseite der Haptik angeordnet ist. In einem anderen Ausführungsbeispiel ist die kohärente Lichtquelle in die Haptik innen liegend integriert. Dies bedeutet in einem Ausführungsbeispiel, dass sie vollständig eingebettet in das Material der Haptik ausgebildet ist. Die kohärente Lichtquelle kann in einem Ausführungsbeispiel vollständig von dem Material der Haptik umschlossen sein. Damit ist die kohärente Lichtquelle in einem Ausführungsbeispiel nicht durch Umgebungseinflüsse, beispielsweise Flüssigkeiten im Auge, beeinträchtigt. Eine dauerhaft hohe Funktionalität ist dadurch ermöglicht.

In einem Ausführungsbeispiel weist die künstliche Augenlinse zumindest ein Lichtleitelement auf. Dieses ist bestimmungsgemäß dazu vorgesehen, dass es Licht der kohärenten Lichtquelle zu einem Display der Bildprojektionsvorrichtung leitet. Mit dem Display wird die optische Information abgestrahlt. Damit ist es ermöglicht, dass die kohärente Lichtquelle und das Display der Bildprojektionsvorrichtung, die grundsätzlich zwei verschiedene Komponenten der Bildprojektionsvorrichtung sind, an unterschiedlichen Orten der künstlichen Augenlinse angeordnet werden können. Dies hat Vorteile bezüglich der positionellen Anordnung sowie einerseits der Erzeugung des Lichts der Bildprojektionsvorrichtung, andererseits der Abstrahlung der optischen Information der Bildprojektionsvorrichtung.

In einem Ausführungsbeispiel weist die Bildprojektionsvorrichtung ein holografisches Display auf. Damit ist es besonders vorteilhaft und einfach ermöglicht, vielfältigste und auch komplexe Hologramme zu erzeugen und in die entsprechende Projektionsrichtung zu projizieren. In einem Ausführungsbeispiel ist dieses Display, insbesondere das holografische Display, an dem optischen Teil angeordnet. Dies ist dahingehend vorteilhaft, da somit der von dem Display emittierte Strahlengang, nämlich die emittierte optische Information direkt auf die Rückseite gestrahlt werden kann. Es sind in dem Zusammenhang somit keine weiteren optischen Elemente erforderlich. Damit kann eine sehr exakte lichtstarke Projektion der optischen Informationen auf die Rückseite, insbesondere über die Rückseite hindurch, nach hinten aus der künstlichen Augenlinse heraus, insbesondere auch ohne Umlenkung der Lichtstrahlen, erfolgen.

Gerade dann, wenn die Lichtquelle der Bildprojektionsvorrichtung eine kohärente Lichtquelle ist, ist dieses kohärente Licht besonders geeignet, ein Hologramm zu erzeugen. Daher ist genau die Kombination zwischen einer kohärenten Lichtquelle und dem holografischen Display besonders vorteilhaft.

Gerade dann, wenn diese beiden verschiedenen Komponenten der Bildprojektionsvorrichtung an unterschiedlichen Orten an oder in der künstlichen Augenlinse angeordnet sind, ist in einem Ausführungsbeispiel zumindest ein Lichtleiterelement der künstlichen Augenlinse vorgesehen. Damit kann gezielt und mit geringen Lichtverlusten das von der kohärenten Lichtquelle emittierte Licht zu dem Display, insbesondere dem holografischen Display, geleitet werden. Unerwünschte Lichtverluste im Material der künstlichen Augenlinse können dann zumindest deutlich reduziert werden. Es kann vorgesehen sein, dass das Lichtleiterelement materiell unterschiedlich zu dem Material der Haptik und/oder dem Material des optischen Teils der künstlichen Augenlinse ist. Damit kann ein ganz bedarfsgerechtes Lichtleiterelement bereitgestellt werden. Es kann in dem Zusammenhang besonders abgestimmt auf das kohärente Licht der kohärenten Lichtquelle ausgebildet sein. Damit kann eine sehr verlustarme Lichtleitung des kohärenten Lichts zu dem Display erfolgen.

Das Lichtleiterelement kann in einem Ausführungsbeispiel stabförmig oder jedoch auch als entsprechende Lichtleiterschicht ausgebildet sein. Möglich ist es in einem Ausführungsbeispiel, dass das Lichtleiterelement in das Material der Haptik und/oder das Material des optischen Teils zumindest bereichsweise eingebettet ist. Es kann jedoch auch vorgesehen sein, dass das Lichtleiterelement vollständig von dem Material der Haptik und/oder vollständig von dem Material des optischen Teils umgeben ist. Auch dadurch ist eine besonders geschützte Anordnung erreicht. In einem Ausführungsbeispiel kann auch vorgesehen sein, dass das Display, insbesondere das holografische Display, zumindest bereichsweise eingebettet in das Material des optischen Teils angeordnet ist. Es kann auch vollständig von dem Material des optischen Teils umschlossen sein.

In einem Ausführungsbeispiel weist die künstliche Augenlinse eine Lochblende auf. Diese ist am oder in dem optischen Teil angeordnet. Dies bedeutet, dass die Lochblende an einer Oberfläche, beispielsweise der Vorderseite des optischen Teils, angeordnet sein kann. Möglich ist es jedoch auch, dass diese Lochblende integriert und in dem Zusammenhang auch vollständig innenliegend in dem Material des optischen Teils ausgebildet ist. Die Lochblende ist vorzugsweise als Ring, insbesondere als Ringscheibe oder Ringplatte, ausgebildet. Sie kann semitransparent oder auch opak ausgebildet sein. Möglich ist es auch, dass das Material der Lochblende in ihrer Transparenz veränderbar ist. Insbesondere ist die Lichtdurchlässigkeit der Lochblende kleiner, insbesondere deutlich kleiner, als die Lichtdurchlässigkeit des Materials des optischen Teils. Diesbezüglich ist das Material des optischen Teils aus einem üblichen Polymermaterial, wie es bei herkömmlichen Linsen, wie beispielsweise Intraokularlinsen oder Kontaktlinsen, Verwendung findet. Hierzu sind vielfältigste Polymermaterialien bekannt, die hier nicht nochmals umfänglichst aufgezählt werden sollen.

In einem Ausführungsbeispiel ist im Flächenbereich der Lochblende hinter der Lochblende zumindest eine elektronische Komponente im optischen Teil angeordnet. Die elektronische Komponente kann beispielsweise eine Antenne sein. Diese elektronische Komponente ist daher zwischen der Lochblende und der Rückseite des optischen Teils angeordnet. Im Flächenbereich der Lochblende kann in einem Ausführungsbeispiel hinter der Lochblende zumindest ein Display, insbesondere ein holografisches Display, der künstlichen Augenlinse im optischen Teil angeordnet sein. Dies bedeutet, dass bei einer Projektionsbetrachtung entlang einer optischen Hauptachse des optischen Teils, der die Vorderseite und die Rückseite des optischen Teils mittig durchdringt, das Display und/oder die elektronische Komponente, insbesondere vollständig, innerhalb des Flächenbereichs der Lochblende angeordnet sind. Diese Positionierung ist dahingehend vorteilhaft, dass der Bereich, der durch die Lochblende flächenmäßig bedeckt ist, ohnehin für einen Lichtdurchlass des Umgebungslichts nicht oder nur eingeschränkt genutzt werden soll und daher eine derartige spezifische Positionierung der elektronischen Komponente und/oder des Displays nicht nachteilig bezüglich der optischen Abbildungseigenschaft des optischen Teils ist.

Diese genannten zusätzlichen Komponenten der künstlichen Augenlinse sind somit quasi verdeckt hinter der Lochblende angeordnet. Auf die Vorderseite des optischen Teils einfallendes Umgebungslicht ist in dem Zusammenhang im Flächenbereich der Lochblende am Durchtritt gehindert oder eingeschränkt.

Durch eine derartige Lochblende kann die künstliche Augenlinse mit verbesserter Funktionalität bezüglich der Schärfentiefe ausgebildet werden.

In einem Ausführungsbeispiel ist die Lochblende zumindest bereichsweise als Energieerzeugungs- und abgabeeinheit zur Erzeugung und zur Abgabe von elektrischer Energie ausgebildet. Damit ist es ermöglicht, dass die künstliche Augenlinse selbst quasi eine elektrische Energieeinheit aufweist. Damit können beispielsweise das Display und/oder die Lichtquelle und/oder weitere, mit elektrischer Energie zu versorgende Komponenten der künstlichen Augenlinse mit derartiger elektrischer Energie versorgt werden. Die Lochblende kann beispielsweise zumindest bereichsweise als Solarzelle ausgebildet sein. Dies ist besonders vorteilhaft, da somit einfallendes Sonnenlicht genutzt werden kann, um daraus elektrische Energie in der künstlichen Augenlinse selbst zu erzeugen.

In einem Ausführungsbeispiel ist die Lochblende vollständig innenliegend im optischen Teil angeordnet. Vorzugsweise ist die vorhandene Lochblende benachbart zu der Vorderseite des optischen Teils an oder in dem optischen Teil angeordnet.

In einem Ausführungsbeispiel kann der optische Teil einen radial äußeren Ringabschnitt aufweisen. Dieser kann vollständig umlaufend ausgebildet sein. Er kann in einem Ausführungsbeispiel nicht zur optischen Abbildungseigenschaft des optischen Teils beitragend ausgebildet sein. An diesem Peripheriering kann in einem Ausführungsbeispiel die Lichtquelle, insbesondere die kohärente Lichtquelle der Bildprojektionsvorrichtung, angeordnet sein. Zusätzlich oder anstatt dazu kann in einem derartigen Peripheriering zumindest eine Antenne der künstlichen Augenlinse angeordnet sein. In diesem Peripheriering kann auch zumindest eine Solarzelle der künstlichen Augenlinse angeordnet sein.

Eine Solarzelle der künstlichen Augenlinse kann eine organische Solarzelle sein. Diese kann dann aus halbleitenden Polymeren oder Molekülen ausgebildet sein. Beispielsweise kann hier Polythiophen, insbesondere Poly(3-Hexylthiophen), das auch als P3HT bezeichnet wird, vorgesehen sein. Dies ist jedoch nur eine beispielhafte und nicht abschließend zu verstehende Nennung eines derartigen Materials. Es sind vielfältigste andere derartige Polymere und Moleküle, die eine organische Solarzelle bilden, möglich. Ebenso können beispielsweise Schichten aus Keramik, wie Perowskiten, als Bestandteil einer Solarzelle vorgesehen sein. Ebenso können aber auch verschiedene Schichten beziehungsweise halbleitende organische Materialien miteinander kombiniert werden, um die Effizienz der Solarzelle zu erhöhen. In dem Zusammenhang können zwei oder mehr Schichten vorgesehen sein, die die Solarzelle bilden. In einem Ausführungsbeispiel können derartige Schichten einer organischen Solarzelle im Bereich zwischen 80 nm und 120 nm, insbesondere zwischen 90 nm und 110 nm, sein. Dadurch lassen sich auch sehr dünne Solarzellen bereitstellen.

Möglich ist es, dass eine Solarzelle durch Kleben oder Drucken auf dem Material des optischen Teils und/oder auf dem Material der Lochblende, wenn eine derartige vorhanden ist, angeordnet ist. Insbesondere ist diesbezüglich die Solarzelle direkt auf dem Material der Lochblende aufgebracht. Damit ist es auch ermöglicht, dass kurze Wege vom elektrischen Verbraucher, insbesondere einem Display, zur Solarzelle erreicht sind. Insbesondere ist dies dann vorteilhaft, wenn das Display unmittelbar benachbart oder gegebenenfalls sogar an der Solarzelle direkt anschließend im optischen Teil angeordnet ist. Insbesondere dann, wenn die Solarzelle vollständig eingebettet und von dem Material des optischen Teils umgeben ist, ist dies vorteilhaft. Denn dann kann die Solarzelle vor Sauerstoff und Wasser geschützt werden.

Das holografische Display kann in einem Ausführungsbeispiel aus Flüssigkristallschichten aufgebaut sein. Eine vorteilhaft vorhandene Antenne kann beispielsweise hinter der Solarzelle und somit auf der der Rückseite des optischen Teils zugewandten Seite der Solarzelle angebracht sein oder aber auch auf einer gegebenenfalls vorhandenen Haptik der künstlichen Augenlinse angeordnet sein. Bei einer Anordnung an der Haptik ist ein besonders störungsfreier Empfang von Signalen, insbesondere Funksignalen, ermöglicht. In einem Ausführungsbeispiel ist das holografische Display hinter der Lochblende, insbesondere hinter einer gegebenenfalls vorhandenen Solarzelle, angeordnet. Hinter bedeutet in dem Zusammenhang, dass das holografische Display auf der der Rückseite des optischen Teils zugewandten Seite der Lochblende angeordnet ist. Eine in dem Zusammenhang hintere Anordnung bedeutet auch, dass entlang der optischen Hauptachse betrachtet dieses holografische Display näher zur Rückseite des optischen Teils positioniert ist, als die Lochblende und/oder die Solarzelle. Damit kann die Lichtabstrahlung von optischen Informationen von dem holografischen Display in Richtung der Rückseite direkt und unbeeinträchtigt erfolgen, da ansonsten keine weiteren Komponenten im Strahlengang von dem Display zur Rückseite angeordnet sind.

Dadurch ist auch eine weitere Vignettierung des einfallenden Lichts verhindert.

In einem Ausführungsbeispiel ist es vorgesehen, dass eine drahtgebundene Verbindung zwischen der Solarzelle und elektrischen Verbrauchern der künstlichen Augenlinse, wie beispielsweise dem holografischen Display und/oder der Lichtquelle, vorgesehen ist. Die diesbezüglich elektrischen Leitungen, insbesondere auch zu Elektroden, sind entsprechend in dem Material des optischen Teils und/oder der Haptik verlegt, insbesondere eingebettet darin angeordnet.

In einem Ausführungsbeispiel ist es möglich, dass die künstliche Augenlinse, insbesondere mittels zumindest einer Antenne, zur drahtlosen Kommunikation mit einer linsenexternen Einheit ausgebildet ist. Diese Kommunikation kann in einem Ausführungsbeispiel bidirektional ausgebildet sein. Damit weist die künstliche Augenlinse auch eine drahtlose Kommunikationsschnittstelle auf. Damit können Informationen, wie beispielsweise auch ein zu erzeugendes Hologrammmuster, drahtlos an die künstliche Augenlinse übertragen werden. Möglich ist es in dem Zusammenhang dann auch, dass Informationen der künstlichen Augenlinse und/oder von einer ihrer integrierten elektrischen und/oder optischen Komponenten über diese drahtlose Kommunikationsschnittstelle von der künstlichen Augenlinse an eine externe Einheit übertragen werden.

In einem Ausführungsbeispiel kann dieses Display, insbesondere das holografische Display, der Bildprojektionsvorrichtung eine Geometrie aufweisen, die der Geometrie einer vorteilhaft vorhandenen Lochblende entspricht. Beispielsweise kann das Display eine Ringform aufweisen. Diese Ringform kann so angeordnet sein, dass sie bei einer Projektionsbetrachtung in Richtung der optischen Hauptachse der künstlichen Augenlinse vollständig von der Fläche der Lochblende verdeckt ist. Weist die künstliche Augenlinse einen Peripheriering an dem optischen Teil auf, so kann die diesbezügliche vorteilhafte Ringgeometrie des Displays auch in dem Zusammenhang bei einer Projektionsbetrachtung im Flächenbereich dieses Peripherierings angeordnet sein. Auch hier kann dann eine Ringform des Displays vorgesehen sein. Insbesondere sind in einem Ausführungsbeispiel die Flächengrößen der Solarzelle und/oder des Displays maximal der Flächengröße der Lochblende und/oder des Peripherierings. Ein diesbezügliches darüber Hinausstehen bei einer entsprechenden Projektionsbetrachtung ist daher in dem Ausführungsbeispiel nicht vorgesehen.

Es kann vorgesehen sein, dass die Flächengröße und Flächenform des Displays und/oder der Solarzelle gleich der Flächenform und der Flächengröße der Lochblende sind, wenn eine solche vorhanden ist. Es kann vorgesehen sein, dass die Flächengröße und Flächenform des Displays und/oder der Solarzelle gleich der Flächenform und der Flächengröße des Peripherierings, wenn ein derartiger vorhanden ist.

In einem Ausführungsbeispiel kann eine Lichtquelle der Bildprojektionsvorrichtung auch eine organische Leuchtdiode aufweisen. Beispielsweise kann sie auch als organische Leuchtdiodenfolie ausgebildet sein.

In einem Ausführungsbeispiel kann ein binäres Amplitudenhologramm als Hologrammmuster erzeugt und durch die Bildprojektionsvorrichtung abgestrahlt werden. Binäre Amplitudenhologramme bestehen aus einem durch einen Computer beziehungsweise eine Recheneinheit berechnetes Muster aus opaken und lichtdurchlässigen Bereichen. Diese lassen sich beispielsweise über ein holografisches Display, welches beispielsweise ein Flüssigkristalldisplay sein kann, oder durch sogenannte smart glasses (Datenbrillen) realisieren, bei denen die Durchlässigkeit mittels angelegter elektrischer Spannung variiert werden kann. Die kohärente Lichtquelle der Bildprojektionsvorrichtung, insbesondere ein Halbleiterlaser, wird danach zur kohärenten Illumination des so erzeugten Hologramms benutzt, um projiziert ein holografisches Bild auf die Rückseite der künstlichen Augenlinse, insbesondere über die Rückseite definiert nach hinten hinaus, zu erzeugen.

In einem Ausführungsbeispiel kann die künstliche Augenlinse auch zumindest einen Sensor aufweisen, mit dem ein Augendruck erfassbar ist. Auch dieser Sensor kann dann mit entsprechender elektrischer Energie versorgt werden und/oder über zumindest eine Antenne der künstlichen Augenlinse Informationen empfangen oder senden. Zusätzlich oder anstatt dazu kann auch ein Sensor zur Überwachung einer Muskeltätigkeit des Auges an der künstlichen Augenlinse vorgesehen sein.

In einem Ausführungsbeispiel weist die künstliche Augenlinse eine Steuereinheit auf. Dies kann Bestandteil der Bildprojektionsvorrichtung sein. Die Steuereinheit kann eine Recheneinheit und einen Speicher aufweisen. Sie kann einen Mikroprozessor aufweisen.

In einem Ausführungsbeispiel kann die künstliche Augenlinse auch einen Positionserfassungssensor aufweisen. Dies kann beispielsweise ein GNSS-(Globales Navigationssatellitensystem)-Sensor sein.

Damit kann eine Positionserkennung der künstlichen Augenlinse erfolgen. Damit kann auch die Position der Person erfasst werden.

In einem Ausführungsbeispiel kann die künstliche Augenlinse mit zumindest einer daran angeordneten Antenne drahtlos mit externen Einheiten kommunizieren. Eine derartige externe Kommunikationseinheit kann beispielsweise ein tragbares Kommunikationsgerät sein. Ein solches Kommunikationsgerät kann beispielsweise ein Mobilfunkendgerät, wie beispielsweise ein Smartphone oder ein Tablet sein. Insbesondere können dadurch auch Informationen von diesem Kommunikationsendgerät zur künstlichen Augenlinse übertragen werden. Auch eine Übertragung von Informationen von der künstlichen Augenlinse zu diesem Kommunikationsendgerät ist dann möglich.

In einem Ausführungsbeispiel kann die Bildprojektionsvorrichtung Bestandteil eines Navigationssystems der künstlichen Augenlinse sein. Somit kann auch ein Navigationssystem an oder in dieser künstlichen Augenlinse angeordnet sein.

Die optischen Informationen, die von der Bildprojektionsvorrichtung erzeugt werden, können beispielsweise auch Handlungsinformationen oder Erinnerungsinformationen sein. Damit können auch Handlungsabläufe durch diese optischen Informationen für eine Person, die die künstliche Augenlinse aufweist, angezeigt und diesbezüglich optisch projiziert werden. Durch Erinnerungsinformationen kann die Person durch die optischen Informationen auch an Termine oder Handlungsabläufe beziehungsweise Vorgehensweisen, die zu erledigen sind und/oder wie derartige Handlungen oder Vorgehensweisen zu erledigen sind, erinnert oder angeleitet werden.

Bei digitalen holografischen 3D-Displays kommen Systeme zum Einsatz, die in der Lage sind, computergesteuert beliebige Hologramme zu erzeugen, um wechselnde holografische Bilder oder auch Videos darzustellen. Diese auch Holo-Displays bezeichneten Systeme zeigen für jede mögliche Betrachtungsrichtung die entsprechende Perspektive, weshalb zur räumlichen Wahrnehmung auch kein optisches Hilfsmittel, wie etwa eine 3D-Brille, nötig ist. Betrachter können ihre Augen auf beliebige Punkte des holografischen Bildes ausrichten und scharfstellen.

Holografische 3D-Displays bieten die Möglichkeit, ein besonderes visuelles Erlebnis zu erzeugen. Die scheinbar freischwebenden Hologramme schaffen es leicht, Aufmerksamkeit zu generieren.

Beim Betrachten eines holografischen Displays wird das Gehirn sofort durch die Illusion getäuscht, dass sich ein physisches Objekt oder eine reale Umgebung mit dem dreidimensionalen digitalen Overlay vermischt. Das visuelle Erlebnis sorgt sofort für Emotionen und regt die Sinne dazu an, die Aktivität im Kortex anzuregen, wodurch Betrachter die vermittelten Informationen besser verarbeiten und behalten können.

Ein holografisches Display funktioniert im Wesentlichen in einem Ausführungsbeispiel dadurch, dass beispielweise ein hochauflösender oder 4K-Bildschirm digitale Inhalte durch Glas mit einer speziellen Beschichtung, der so genannten Glasoptik, reflektiert. Wenn die Glasoptik in einem bestimmten Winkel platziert wird, erzeugt sie eine Illusion, die das Gehirn dazu bringt, den digitalen Inhalt als dreidimensional zu interpretieren. Dadurch entsteht das Gefühl, ein freischwebendes realistisches Objekt vor den Augen zu haben.

Die meisten Ansätze für Holo-Displays basieren auf sogenannten Spatial Light Modulators (SLM). Das sind Chips mit rasterförmig angeordneten, elektronisch ansteuerbaren Pixeln. Am häufigsten finden sogenannte Flüssigkristall-SLM Verwendung.

Ein weiterer unabhängiger Aspekt der Erfindung betrifft ein elektronisches Informationssystem mit einer künstlichen Augenlinse. Diese künstliche Augenlinse kann in einem Ausführungsbeispiel gemäß einem oben genannten Aspekt oder einem vorteilhaften Ausführungsbeispiel davon ausgebildet sein. Das elektronische Informationssystem weist darüber hinaus zumindest ein Kommunikationsgerät auf. Dieses Kommunikationsgerät ist ein zur künstlichen Augenlinse separate und extern zur künstlichen Augenlinse angeordnetes Gerät. Das Kommunikationsgerät kann ein einziges Gerät sein oder ein Kommunikationssystem sein. Darüber hinaus weist das elektronische Informationssystem eine drahtlose Kommunikationsschnittstelle auf, mit welcher eine Übertragung von Informationssignalen zwischen der künstlichen Augenlinse und dem Kommunikationsgerät durchführbar ist. Durch ein derartiges elektronisches Informationssystem kann somit eine Kommunikation zwischen einer künstlichen Augenlinse, wie beispielsweise einer Intraokularlinse, mit einem linsenexternen Kommunikationsgerät erfolgen. Damit kann insbesondere dann, wenn die künstliche Augenlinse am oder im Auge angeordnet ist, eine Kommunikation, die insbesondere auch bidirektional sein kann, mit einem linsenexternen Kommunikationsgerät erfolgen. Damit kann in dem Zusammenhang auch eine Signalübertragung zu einem Kommunikationsgerät oder von einem Kommunikationsgerät erfolgen, welches extern zu einer Person, die die künstliche Augenlinse am oder im Auge aufweist, angeordnet ist. Durch ein derartiges elektronisches Informationssystem können medizinische Informationen vom Auge und/oder der künstlichen Augenlinse dann, wenn die künstliche Augenlinse an oder im Auge angeordnet ist, zu dem Kommunikationsgerät übertragen werden. Damit können auch beispielweise auch medizinische Zustände des Auges und/oder der künstlichen Augenlinse durch das elektronische Informationssystem ausgewertet werden. Möglich ist es auch, dass abhängig von diesen Zuständen und/oder auch abhängig von anderen Parametern, beispielsweise Umgebungseinflüssen auf die Person mit dem Auge und der künstlichen Augenlinse und/oder abhängig von der Position der Person mit der künstlichen Augenlinse, Informationen von dem Kommunikationsgerät zur künstlichen Augenlinse übertragen werden. Diese Informationen können Handlungsinformationen und/oder Erinnerungsinformationen sein. Beispielsweise können diese Informationen über einen Weg, den die Person nehmen soll, sein. Es können daher Navigationsinformationen sein.

Möglich ist es auch, dass Informationen zur Erinnerung an einen Termin übertragen werden. Ebenso können Informationen bezüglich eines Zeitpunkts und/oder einer Menge einer Medikamenteneinnahme übertragen werden. Ebenso können jedoch auch Informationen übertragen werden, die unabhängig von medizinischen Aspekt Handlungsweisen oder Vorgehensweisen der Person beinhalten. Beispielsweise können dies Informationen zur Bedienung eines Haushaltsgerätes sein. Ebenso können diesbezüglich Bedienungsinformation für anderweitige Geräte, wie beispielsweise eine elektronische Parkuhr, einen Fahrkartenautomat oder sonstige, in der Umgebung angeordnete und öffentlich zugänglich und bedienbare elektronische Geräte sein. Damit kann hilfsbedürftigen Personen auf Anforderung oder auch automatisch durch dieses elektronische Informationssystem zumindest eine Unterstützung erbracht werden.

Insbesondere für hilfsbedürftige Personen, beispielsweise ältere Personen und/oder körperlich und/oder geistig beeinträchtigte Personen können dadurch unterstützt werden. Insbesondere wenn das elektronische System auch dazu ausgebildet ist, automatisch die jeweilige Hilfssituation für die Person zu erkennen, kann auch dann automatisch der Zeitpunkt für die diesbezügliche Informationsbereitstellung und/oder die Art und/oder Mengel der entsprechenden Informationsbereitstellung durch das elektronische Informationssystem erstellt und bereitgestellt werden. Gerade durch ein derartiges elektronisches Informationssystem ist es dann auch ermöglicht, dass die Person mit dem Auge und der künstlichen Augenlinse das Kommunikationsgerät gar nicht mehr selbst mitführen muss. Durch die künstliche Augenlinse, die ohnehin von der Person am oder im Auge getragen wird, kann auch einfach und schnell und unvermittelt durch die Person wahrnehmbar die entsprechende Information dargeboten werden. So ist es in dem Zusammenhang auch möglich, dass durch das Kommunikationsgerät zumindest ein elektrisches Signal über die Kommunikationsschnittstelle übertragen wird und durch eine Bildprojektionsvorrichtung, die am oder in der künstlichen Augenlinse angeordnet ist und Bestandteil der künstlichen Augenlinse ist, in eine optische Information umgewandelt wird. Diese optische Information wird durch die Bildprojektionsvorrichtung dann in gezielter und definierter Weise vorzugsweise in eine ganz spezifische Projektionsrichtung abgestrahlt. Die Projektionsrichtung ist in einem Ausführungsbeispiel direkt auf die Rückseite der künstlichen Augenlinse, insbesondere des optischen Teils der künstlichen Augenlinse gerichtet.

Vorzugsweise ist diese Projektionsrichtung so gestaltet, dass zumindest ein Fokus hinter der künstlichen Augenlinse, insbesondere hinter dem optischen Teil der künstlichen Augenlinse ist. Damit wird es gezielt ermöglicht, dass die Information von dem Kommunikationsgerät über die drahtlose Kommunikationsschnittstelle an die künstliche Augenlinse übertragen wird und dort durch die Bildprojektionsvorrichtung in eine optische Information gewandelt wird, die dann in ganz spezifischer Projektionsrichtung direkt auf die Netzhaut des Auges projiziert wird.

In einem Ausführungsbeispiel kann die künstliche Augenlinse eine Steuereinheit aufweisen. Diese kann eine elektronische Recheneinheit, wie beispielsweise ein Mikroprozessor sein. Die Steuereinheit kann auch eine Speichereinheit aufweisen. Die Steuereinheit kann in einem Ausführungsbeispiel am oder im optischen Teil der künstlichen Augenlinse angeordnet sein. Weist die künstliche Augenlinse eine Lochblende auf, kann diese Steuereinheit bezüglich einer Flächenprojektionsbetrachtung im Flächenbereich der Lochblende angeordnet sein. Weist der optische Teil einen radial äußeren Peripheriering auf, der insbesondere nicht zur optischen Abbildungseigenschaft des optischen Teils beiträgt, kann die Steuereinheit auch in oder an diesem Peripheriering angeordnet sein. Möglich ist es in einem Ausführungsbeispiel auch, dass diese Steuereinheit, wenn sie an der künstlichen Augenlinse angeordnet oder integriert darin angeordnet ist, in einer oder an einer Haptik der künstlichen Augenlinse angeordnet ist, wenn die künstliche Augenlinse einer derartige Haptik aufweist.

In einem anderen Ausführungsbeispiel ist es auch möglich, dass die Steuereinheit extern zur künstlichen Augenlinse angeordnet ist. Beispielsweise kann sie in dem Kommunikationsgerät angeordnet sein. Durch die Steuereinheit ist es in einem Ausführungsbeispiel ermöglicht, den Zeitpunkt des Sendens einer Information von dem Kommunikationsgerät zur künstlichen Augenlinse oder das Senden einer Information von der künstlichen Augenlinse zum Kommunikationsgerät zu steuern. Darüber hinaus kann durch die Steuereinheit auch die Art und die Menge der jeweils zu übertragenden Informationen gesteuert werden. In einem Ausführungsbeispiel ist es auch möglich, dass die Steuereinheit dazu ausgebildet ist, die Bildprojektionsvorrichtung der künstlichen Augenlinse entsprechend zu steuern. Damit kann sowohl das Aktivieren und Deaktivieren der Bildprojektionsvorrichtung als auch die Zeitdauer des aktiven Zustands der Bildprojektionsvorrichtung gesteuert werden. Ebenso ist es durch die Steuereinheit ermöglicht, dass die zu projizierenden optischen Informationen entsprechend ausgewählt und projiziert werden. In einem Ausführungsbeispiel kann durch die Steuereinheit die Laserdiode der Bildprojektionsvorrichtung und/oder das Display der Bildprojektionsvorrichtung entsprechend gesteuert werden.

In einem anderen Ausführungsbeispiel kann zusätzlich oder anstatt dazu das Aktivieren des elektronischen Informationssystems auch durch eine Person, die die künstliche Augenlinse im oder am Auge trägt, erfolgen. Beispielsweise kann dies durch eine definierte Handlungsweise erfolgen. Möglich ist es in dem Zusammenhang, dass das Lid für eine vorgegebene Zeitdauer geschlossen wird. Dieser Dunkelzustand kann durch die künstliche Augenlinse erkannt werden und als nutzerinitiiertes Kommando zum Aktivieren oder Deaktivieren der Bildprojektionsvorrichtung erkannt werden. Ebenso ist es möglich, dazu das Auge mit einer Hand zuzuhalten.

Möglich ist es jedoch auch, dass die Person mit der künstlichen Augenlinse beispielsweise ein Kommunikationsendgerät, wie beispielsweise ein tragbares Kommunikationsendgerät, wie beispielsweise ein Smartphone oder ein Tablet aufweist. Dieses Kommunikationsgerät kann Bestandteil des elektronischen Informationssystems sein. Der Nutzer beziehungsweise die Person, die die künstliche Augenlinse am oder im Auge trägt, kann dann mit diesem Kommunikationsgerät ebenfalls die oben genannten Abläufe initiieren. Beispielsweise kann dies durch manuelle Eingabe in das Kommunikationsgerät erfolgen. Möglich ist jedoch auch eine Spracheingabe.

Darüber hinaus ist es jedoch auch möglich, dass das Kommunikationsgerät ein nutzerunabhängiges Gerät ist. Dies bedeutet, dass es nicht von der Person, die die künstliche Augenlinse im oder am Auge trägt, mitgeführt ist. Damit kann das Kommunikationsgerät beispielsweise von einer Hilfsperson benutzt oder bedient werden, die diejenige andere Person, die die künstliche Augenlinse am oder im Auge trägt, diesbezüglich unterstützt. Beispielsweise kann die Person medizinisches Hilfspersonal oder beispielsweise ein Familienangehöriger oder dergleichen sein. Damit kann insbesondere älteren Personen und/oder körperlich oder geistig beeinträchtigten Personen und/oder im Sehvermögen beeinträchtigen Personen diesbezüglich eine entsprechende Unterstützung zu Teil werden. Gerade bei geistig beeinträchtigen Personen, wie beispielsweise dementen Personen, ist dieses elektronische Informationssystem besonders vorteilhaft sein.

Es kann dann nicht nur damit im normalen Alltag eine entsprechende elektronische Unterstützung durch dieses elektronische System erfolgen. Sowohl in dem eigenen Wohnbereich, als auch extern dazu kann eine systemseitige Unterstützung erfolgen. So kann beispielsweise ein Verirren einer derartigen Person auch außerhalb des Wohnbereichs ebenso besser verhindert werden, als auch das Durchführen von unerwünschte und/oder sicherheitskritischen Handlungen im Wohnbereich besser vermieden werden. Beispielsweise kann in dem Zusammenhang auch ein unerwünschtes aktiviert sein eines Haushaltsgeräts, wie beispielsweise eines Kochfelds oder eines Backofens, oder eines Wasserhahns oder einer Dusche oder eines Wassereinlaufs in eine Badewanne oder dergleichen besser vermieden werden. So können in dem Zusammenhang auch derartige Zustände dahingehend besser vermieden werden, dass mit dem elektronischen Informationssystem Informationen bereitgestellt werden, die ein Beenden oder Deaktivieren derartiger Geräte im Haushalt darbietet. Diesbezüglich können dann optische Informationen mit der Bildprojektionsvorrichtung auf die Netzhaut der entsprechenden Person projiziert werden.

Insbesondere sind optische Informationen derartige, die von der Person, die die künstliche Augenlinse im Auge trägt, zum Zeitpunkt der Informationserzeugung und/oder zum Zeitpunkt der Informationsprojektion nicht selbst in der Umgebung wahrgenommen werden können und/oder zwar wahrgenommen werden können aber die Situation und/oder die daraus sich ergebenden Konsequenzen nicht erkannt werden können.

Möglich ist es durch das elektronische Informationssystem auch, dass abhängig von einer Position der künstlichen Augenlinse und somit von der Position derjenigen Person, die diese künstliche Augenlinse am oder im Auge trägt und/oder abhängig von einem aktuellen Aufenthaltsbereich dieser Person und/oder abhängig von der Zeitdauer eines Entfernens von einem spezifischen Ort und/oder abhängig von einer Zeitdauer, die bezüglich eines Ankommens zu einem bestimmten Zeitpunkt an einem Ort, überschritten wird, automatisch eine entsprechende Information durch das elektronische Informationssystem erzeugt wird und als optische Information auf die Netzhaut einer Person mit der künstlichen Augenlinse projiziert wird.

In dem Zusammenhang kann das elektronische Informationssystem auch als elektronisches Netzhaut-Informationsdarstellungssystem bezeichnet werden. Insbesondere können optische Informationen derartige sein, die zusätzlich zu den Informationen, die aktuell durch Betrachtung der Umgebung mit dem Auge selbst wahrgenommen werden können, angezeigt werden. Insbesondere sind diese optischen Informationen daher solche, die aktuell durch Betrachtung der Umgebung mit den Augen einer Person nicht wahrgenommen werden können.

Möglich ist es auch, dass die Kommunikationsschnittstelle eine Funkschnittstellte ist.

In einem weiteren Ausführungsbeispiel kann das elektronische Informationssystem maschinell trainiert werden. Insbesondere können in dem Zusammenhang Verhaltensweisen der Person aus der Vergangenheit ausgewertet werden und diesbezüglich zukünftige Informationserzeugungen und/oder Informationsdarstellungen durch das System selbst angepasst werden. Nicht in dem Zusammenhang können in den einzelnen Situationen auch die Blickrichtung und/oder eine Kopfbewegung einer Person, die die künstliche Augenlinse am oder im Auge trägt, erfasst und ausgewertet werden.

In einem weiteren Ausführungsbeispiel kann vorgesehen sein, dass das elektronische Informationssystem eine potentielle Wahrnehmung und ein entsprechendes Verständnis der projizierten optischen Information durch die Person selbst auswertet. Beispielsweise kann dies dahingehend erfolgen, dass eine unverzügliche Handlung der Person dahingehend erfolgt, die das Verständnis der projizierten optischen Information charakterisiert. Zusätzlich oder anstatt dazu kann beispielsweise auch eine definierte Kopfbewegung ausgewertet werden, die signalisiert, dass die Person die projizierten optischen Information wahrgenommen und verstanden hat. Folgt derartiges nicht, so kann in einem Ausführungsbeispiel durch das elektronische Informationssystem die optische Information in geänderter Art und Weise anzeigen. Beispielsweise kann hier ein Größenänderung und/oder Farbänderung und/oder eine dynamische Anzeige, beispielsweise als Lauflicht oder als Blinklicht erfolgen. Auch diesbezüglich kann dann in einem Ausführungsbeispiel das elektronische Informationssystem sich selbst maschinell trainieren, indem es erkennt, welche dieser Hinweisinformationen in jeweiliger Situation zum schnellen und vollständigen Verständnis durch die Person geführt haben und/oder daraus resultierend eine richtige Handlungsweise erfolgt ist. In dem Zusammenhang kann dann das maschinelle Trainieren eine Prioritätenliste diesbezüglich nach sich ziehen, die durch das elektronische Informationssystem selbstständig erstellt wird.

Ein weiterer Aspekt betrifft ein Verfahren zum Darstellen von Informationen auf einer Netzhaut eines Auges. Bei dem Verfahren werden optische Informationen mit einer Bildprojektionsvorrichtung, die an oder in einer künstlichen Augenlinse, die insbesondere gemäß dem oben genannten unabhängigen Aspekt oder einem vorteilhaften Ausführungsbeispiel davon ausgebildet sein kann, ausgebildet ist, mit einer vorgegebenen Projektionsrichtung direkt zur Rückseite der Augenlinse, insbesondere eines optischen Teils der künstlichen Augenlinse abgestrahlt. Insbesondere derart, dass ein Brennpunkt dieser Projektion außerhalb der Augenlinse hinter der Rückseite liegt, insbesondere die optischen Informationen direkt auf die Netzhaut projiziert werden, wenn die künstliche Augenlinse in dem Auge angeordnet ist.

Vorteilhafte Ausführungsbeispiele des Verfahrens sind durch die oben genannten vorteilhaften Aspekte betreffend die künstliche Augenlinse einerseits und/oder betreffend das elektronische Informationssystem andererseits zu verstehen. Die Komponenten und/oder deren Zusammenwirken ermöglichen die jeweiligen Schritte.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1: eine schematische Draufsicht auf ein Ausführungsbeispiel einer künstlichen Augenlinse;
- Fig. 2: eine schematische Draufsicht auf ein weiteres Ausführungsbeispiel einer erfindungsgemäßen künstlichen Augenlinse; und
- Fig. 3: eine schematische Schnittdarstellung durch ein Ausführungsbeispiel einer erfindungsgemäßen künstlichen Augenlinse mit einer zusätzlichen schematischen Darstellung einer Netzhaut eines Auges.

### Bevorzugte Ausführungsbeispiele der Erfindung

In den Figuren werden gleiche oder funktionsgleiche Elemente mit den gleichen Bezugszeichen versehen.

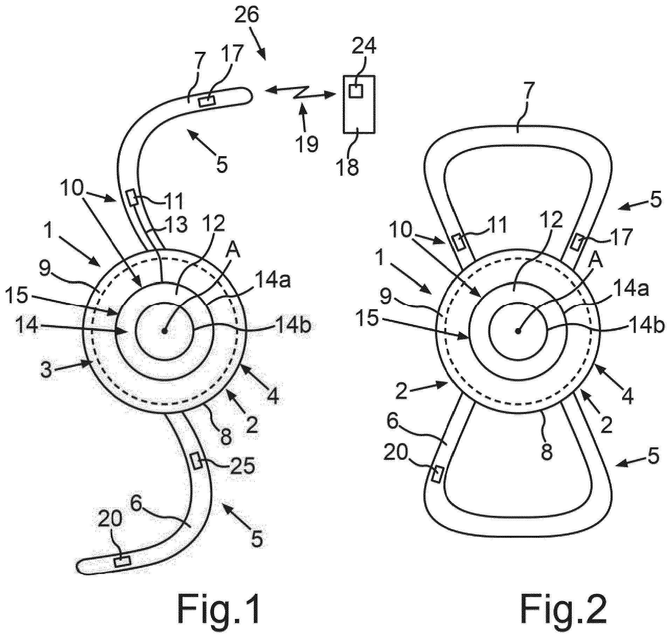

In Fig. 1 ist in einer schematischen Draufsicht ein Ausführungsbeispiel einer künstlichen Augenlinse 1 gezeigt. Die künstliche Augenlinse 1 ist eine Intraokularlinse. Sie weist einen optischen Teil 2 auf. Dieser optische Teil 2 bildet dieses eigentliche optische Abbildungselement der künstlichen Augenlinse 1. Es kann diesbezüglich vielfältig gestaltet sein und weist von der Grundgeometrie eine scheibenartige Form auf. Dieser optische Teil 2 weist eine Vorderseite 3 auf. Die Vorderseite 3 kann konvex oder konkav gekrümmt sein. Die Vorderseite 3 ist bestimmungsgemäß diejenige Seite, die dann, wenn die künstliche Augenlinse 1 im Auge implantiert ist, oder bei einer Ausgestaltung als Kontaktlinse, an der Hornhaut des Auges frontseitig anliegt, der vorderen Umgebung der Person zugewandt ist. Dies bedeutet auch, dass die Vorderseite 3 diejenige Seite ist, die im Auge angeordneten Zustand der künstlichen Augenlinse 1 der Netzhaut, insbesondere dem Sehnerv des Auges abgewandt ist. Der optische Teil 2 weist darüber hinaus eine der Vorderseite 3 gegenüberliegende Rückseite 4 auf. Diese kann konvex oder konkav gekrümmt sein. Die Rückseite 4 ist dann, wenn die künstliche Augenlinse 1 im Auge angeordnet ist, der Netzhaut, insbesondere dem Sehnerv des Auges zugewandt. Der optische Teil 2 weist eine optische Hauptachse A auf, die in Fig. 1 senkrecht zur Figurenebene orientiert ist. Die optische Hauptachse A durchdringt die Vorderseite 3 und die Rückseite 4 mittig.

Im Ausführungsbeispiel weist diese künstliche Augenlinse 1 auch eine Haptik 5 auf. Diese ist im Ausführungsbeispiel durch zwei separate Bügel 6 und 7 gebildet. Diese Bügel 6 und 7 enden jeweils hier mit einem Ende an einer Umfangswand 8 des optischen Teils 2.

Der optische Teil 2 kann über die gesamte Geometrie zur optischen Abbildungseigenschaft beitragen. In einem anderen Ausführungsbeispiel kann vorgesehen sein, dass der optische Teil 2 in radialer Richtung zur optischen Hauptachse A betrachtet einen äußeren Ring 9 aufweist. Dieser kann auch als äußerer Abschlussring oder Peripheriering bezeichnet werden. Dieser Peripheriering, der in Fig. 1 radial nach innen hin symbolisch durch die gestrichelte Linie begrenzt ist, weist keine optische Abbildungseigenschaft auf. Er ist integriert und somit einstückig mit dem restlichen Bereich des optischen Teils 2 ausgebildet. Insbesondere ist der optische Teil 2 aus einem Polymermaterial ausgebildet. Der optische Teil 2 und die Haptik 5 können einstückig ausgebildet sein. Möglich ist es auch, dass die Bügel 6 und 7 jedoch separate Komponenten sind, die eingesteckt oder angeklebt an dem optischen Teil 2 angeordnet sind.

Im Ausführungsbeispiel weist die künstliche Augenlinse 1 auch eine Bildprojektionsvorrichtung 10 auf. Diese ist somit in der künstlichen Augenlinse 1 integriert oder an einer Oberfläche dazu direkt angeordnet. Die Bildprojektionsvorrichtung 10 ist dazu ausgebildet, eine durch die Bildprojektionsvorrichtung 10 selbst erzeugte optische Information zumindest auf die Rückseite 4 des optischen Teils 2 zu projizieren. Insbesondere ist die Bildprojektionsvorrichtung 10 dazu ausgebildet, eine durch die Bildprojektionsvorrichtung 10 erzeugte optische Information durch die Rückseite 4 des optischen Teils 2 hindurch aus dem optischen Teil 2 heraus nach hinten zu projizieren. Es ist diesbezüglich somit ein definiertes Projektionsszenario mit einer definierten Projektionsrichtung vorgegeben. Die Bildprojektionsvorrichtung 10 ist in einem Ausführungsbeispiel zur Erzeugung eines 3D-Bilds ausgebildet. Insbesondere ist sie zur Erzeugung eines Hologramms ausgebildet. Die Bildprojektionsvorrichtung 10 weist in einem Ausführungsbeispiel zumindest eine Lichtquelle 11 auf. Die Lichtquelle kann eine kohärente Lichtquelle 11 sein. Insbesondere kann sie eine Laserdiode, wie beispielsweise ein entsprechender Halbleiterlaser, sein. Diese Lichtquelle 11 ist an der künstlichen Augenlinse 1 selbst angeordnet. Sie kann in einem Ausführungsbeispiel als Lichtquelle 11 realisiert sein, die in der Haptik 5 angeordnet ist. Insbesondere kann sie vollständig in der Haptik 5 angeordnet sein. Sie kann diesbezüglich von dem Material der Haptik 5 vollständig umgeben sein.

In einem anderen Ausführungsbeispiel kann die Lichtquelle 11 in dem Peripheriering 9, wenn dieser vorhanden ist, ausgebildet sein. In einem Ausführungsbeispiel kann die Lichtquelle 11 dann vollständig innerhalb des Materials des Peripherierings 9 angeordnet sein.

Insbesondere ist die Lichtquelle 11 an einer derartigen Position an oder in der künstlichen Augenlinse 1 angeordnet, in welcher sie die optische Abbildungseigenschaft des optischen Teils 2 nicht beeinträchtigt.

Die Bildprojektionsvorrichtung 10 kann in einem Ausführungsbeispiel ein Display 12 aufweisen. Insbesondere ist das Display 12 ein holografisches Display. In einem Ausführungsbeispiel ist das Display 12 am oder vollständig innerhalb des optischen Teils 2 angeordnet. Das Display 12 kann dann, wenn der Peripheriering 9 vorhanden ist, in dem Bereich des Peripherierings 9 angeordnet sein. Zusätzlich oder anstatt dazu kann das Display 12 jedoch auch zumindest bereichsweise radial weiter innen angeordnet sein, wenn der optische Teil 2 den Peripheriering 9 aufweist. Weist der optische Teil 2 den Peripheriering 9 nicht auf, kann das Display 12 ebenfalls an verschiedenen Positionen am oder im optischen Teil 2 angeordnet sein.

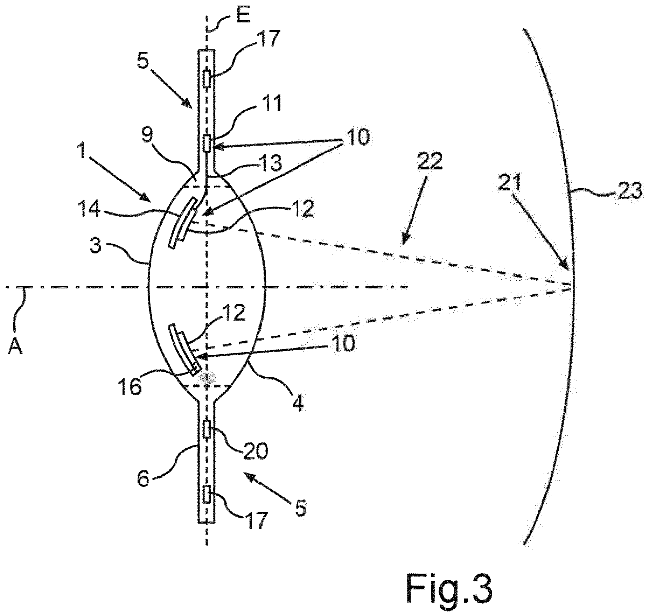

In einem Ausführungsbeispiel, in dem das Display 12 und die Lichtquelle 11 beabstandet zueinander in der künstlichen Augenlinse 1 angeordnet sind, kann die Bildprojektionsvorrichtung 10 ein Lichtleitelement 13 (Fig. 3) aufweisen. Mit diesem Lichtleitelement 13 kann das Licht der Lichtquelle 11 zu dem Display 12 geleitet werden. Das Lichtleitelement 13 ist in einem Ausführungsbeispiel vollständig innenliegend in dem Material der Haptik 5 und/oder des optischen Teils 2 angeordnet.

In einem Ausführungsbeispiel weist die künstliche Augenlinse 1 eine Lochblende 14 auf, wie dies in Fig. 1 gezeigt ist. Die Lochblende 14 ist als Ring ausgebildet. In einem Ausführungsbeispiel kann die Lochblende 14 zumindest bereichsweise als fotoelektrisches Element ausgebildet sein. Ein Beispiel für ein fotoelektrisches Element ist eine Solarzelle. Das fotoelektrische Element 15 kann ebenfalls ringförmig ausgebildet sein. Insbesondere kann in einem Ausführungsbeispiel die Lochblende 14 durch dieses fotoelektrische Element 15 gebildet sein.

In einem Ausführungsbeispiel ist das Display 12 bei einer Flächenprojektionsbetrachtung und somit bei Blick in Richtung der optischen Hauptachse A vollständig in dem Flächenbereich der Lochblende 14 angeordnet. Es kann in einem Ausführungsbeispiel vorgesehen sein, dass das Display 12 in Bezug zu der Vorderseite 3 gesehen hinter der Lochblende 14 angeordnet ist. Dies ist in der Schnittdarstellung in Fig. 3 zu erkennen. In der dortigen schematischen Schnittdarstellung ist auch zu erkennen, dass das Display 12 direkt an der Rückseite der Lochblende 14 angeordnet ist. Insbesondere dann, wenn auch ein fotoelektrisches Element 15 vorhanden ist, kann das Display 12 mit elektrischer Energie durch dieses fotoelektrische Element 15 versorgt werden. Hierzu können entsprechende Elektroden und/oder elektrische Leitungen vorgesehen sein. Das Display 12 kann auch direkt an dem fotoelektrischen Element 15 angeordnet sein. Das Display 12 ist von seiner Flächengröße kleiner oder maximal gleich groß dem Flächenbereich der Lochblende 14. Das Display 12 kann in einem Ausführungsbeispiel die Geometrie der Lochblende 14 aufweisen. Es kann somit auch ringförmig gestaltet sein. Wie in Fig. 1 zu erkennen ist, ist die Lochblende 14 durch einen radial äußeren Rand 14a und einen radial inneren Rand 14b begrenzt.

In Fig. 2 ist in einer schematischen Draufsicht ein weiteres Ausführungsbeispiel einer künstlichen Augenlinse 1 gezeigt. Diese ist im Unterschied zu Fig. 2 lediglich mit einer verschiedenen Haptik 5 ausgebildet. Bei dem Ausführungsbeispiel in Fig. 2 sind die hier auch zwei Bügel 6 und 7 nicht mehr jeweils mit einem Ende an dem optischen Teil 2 angeordnet, sondern jeweils mit beiden Enden an unterschiedlichen Stellen an dem optischen Teil 2 endend.

Im Flächenbereich der Lochblende 14 kann in einem Ausführungsbeispiel hinter der Lochblende 14 zumindest eine elektronische Komponente der künstlichen Augenlinse 1 angeordnet sein. Die elektronische Komponente kann beispielsweise zumindest eine Antenne 16 sein. Die Antenne 16 kann somit auf der der Rückseite 4 zugewandten Seite der Lochblende 14 angeordnet sein. Sie kann vollständig innerhalb des Flächenbereichs der Lochblende 14 angeordnet sein. Zusätzlich oder anstatt dazu kann die künstliche Augenlinse 1 auch eine Antenne 17 (Fig. 1) aufweisen. Die Antenne 17 kann hier in der Haptik 5 angeordnet sein. Mit der Antenne 16 und/oder der Antenne 17 kann eine drahtlose Kommunikation mit einem externen Gerät 18, wie es in Fig. 1 gezeigt ist, erfolgen.

Insbesondere kann eine bidirektionale Kommunikation über die Schnittstelle 19 erfolgen. Entsprechendes ist auch bei dem Ausführungsbeispiel gemäß Fig. 2 und 3 ermöglicht.

In einem Ausführungsbeispiel kann die Solarzelle 15 auch im Flächenbereich des Peripherierings 9 angeordnet sein, wenn ein derartiger ausgebildet ist. Insbesondere kann die Solarzelle 15 dann eine entsprechende Geometrie und/oder eine entsprechende Flächengröße wie der Peripheriering 9 aufweisen.

In einem weiteren Ausführungsbeispiel kann die künstliche Augenlinse 1 auch zumindest einen GNSS-Sensor 20 aufweisen. Dieser kann in der Haptik 5 angeordnet sein.

In Fig. 3 ist, wie bereits oben erläutert, eine schematische Vertikalschnittdarstellung durch ein Ausführungsbeispiel einer künstlichen Augenlinse 1, hier einer Intraokularlinse, gezeigt. Es ist hier die Projektionsrichtung 22 der Bildprojektionsvorrichtung 10 gezeigt. Mit der Bildprojektionsvorrichtung 10 wird ein Bild definiert an dem Display 12 zumindest auf die Rückseite 4, insbesondere durch die Rückseite 4 hindurch, nach hinten außerhalb der künstlichen Augenlinse 1 projiziert. Insbesondere kann mit dieser Bildprojektionsvorrichtung 10 ein Bild auf eine Netzhaut 23, insbesondere auch im Bereich der Makula 21 und/oder des Sehnervs eines Auges erfolgen, in dem die künstliche Augenlinse 1 angeordnet ist. Vorzugsweise ist das Display 12 dazu so in der künstlichen Augenlinse 1 angeordnet, dass im Strahlengang zwischen dem Display 12 und der Rückseite 4 kein weiteres integriertes Element der künstlichen Augenlinse 1 angeordnet ist, sondern lediglich das Material, insbesondere das Polymermaterial, aus dem der optische Teil 2 ausgebildet ist, angeordnet ist.

In einem Ausführungsbeispiel kann die künstliche Augenlinse 1 noch zumindest einen weiteren Sensor aufweisen. Der weitere Sensor kann beispielsweise ein Temperatursensor und/oder ein Drucksensor sein, mit welchem die Temperatur im Auge und/oder der Augendruck, gemessen werden können.

Die künstliche Augenlinse 1 kann in einem Ausführungsbeispiel auch eine Steuereinheit 25 aufweisen.

Das Display 12 kann ein holografisches Flüssigkristalldisplay sein. Es kann aus mehreren Schichten aufgebaut sein. Auch die Solarzelle 15 kann aus einer oder mehreren Schichten aufgebaut sein. Das Lichtleitelement 13 kann in einem Ausführungsbeispiel als Folie ausgebildet sein.

Ein weiterer Aspekt der Erfindung betrifft ein Kommunikationssystem mit einem Ausführungsbeispiel einer künstlichen Augenlinse 1 und einem dazu separaten externen Kommunikationsgerät 18. Das Kommunikationssystem kann ein elektronisches Informationssystem 26 sein, wie es in einem Beispiel in Fig. 1 vereinfacht gezeigt ist. Dieses elektronische Informationssystem 26 weist auch die Kommunikationsschnittstelle 19 auf. Das externe Kommunikationsgerät 18 kann drahtlos über die Kommunikationsschnittstelle 19, die insbesondere eine Funkschnittstelle sein kann, mit der künstlichen Augenlinse 1 kommunizieren. Über diese Kommunikationsschnittstelle 19 kann auch die künstliche Augenlinse 1 mit dem zumindest einen Kommunikationsgerät 18 kommunizieren. In einem Ausführungsbeispiel kann das Kommunikationsgerät 18 eine Steuereinheit 24 aufweisen.

Möglich ist es auch, dass mit dem Kommunikationsgerät 18 Informationen an die künstliche Augenlinse 1 übertragen werden, die dort mit der Bildprojektionsvorrichtung 10 als optische Informationen erzeugt und entsprechend, wie es erläutert wurde, zumindest auf die Rückseite 4, insbesondere durch die Rückseite hindurch, aus dem optischen Teil 2 heraus projiziert werden. Darüber hinaus ist es auch möglich, dass die mit zumindest einem Sensor, der an oder in der künstlichen Augenlinse 1 angeordnet ist, erfassten Informationen über die Kommunikationsschnittstelle 19 an das zumindest eine externe Kommunikationsgerät 18 übertragen werden.

In einem Ausführungsbeispiel kann, wie dies in Fig. 3 zu erkennen ist, die Lochblende 14 in einer vorderen Hälfte des optischen Teils 2, die sich zwischen der Vorderseite und einer Mittenebene E des optischen Teils 2 bildet, angeordnet sein. Insbesondere kann auch das Display 12 zumindest bereichsweise, insbesondere vollständig, in dieser vorderen Hälfte der künstlichen Augenlinse 1 angeordnet sein.

## Patentansprüche

1. Künstliche Augenlinse (1) mit zumindest einem optischen Teil (2), der eine Vorderseite (3) und eine der Vorderseite (3) gegenüberliegende Rückseite (4) aufweist, wobei die künstliche Augenlinse (1) eine Bildprojektionsvorrichtung (10) aufweist,
**dadurch gekennzeichnet, dass**
mit der Bildprojektionsvorrichtung (10) eine durch die Bildprojektionsvorrichtung (10) selbst erzeugte optische Information zumindest auf die Rückseite (4) des optischen Teils (2) projiziert ist, wobei die Bildprojektionsvorrichtung (10) eine Bilderzeugung unabhängig von der optischen Abbildungseigenschaft des optischen Teils (2) ermöglicht, wobei die Bildprojektionsvorrichtung (10) so am oder im optischen Teil (2) angeordnet ist, dass sie das von ihr emittierte Lichtbild direkt zur Rückseite (4) strahlt und die Bildprojektionsvorrichtung (10) dazu ausgebildet ist, eine durch die Bildprojektionsvorrichtung (10) erzeugte optische Information durch die Rückseite (4) des optischen Teils (2) hindurch aus dem optischen Teil (2) heraus nach hinten zu projizieren.

2. Künstliche Augenlinse (1) nach Anspruch 1,
**dadurch gekennzeichnet, dass**
die Bildprojektionsvorrichtung (10) zur Erzeugung eines 3D-Bilds ausgebildet ist.

3. Künstliche Augenlinse (1) nach einem der vorhergehenden Ansprüche,
**dadurch gekennzeichnet, dass**
die Bildprojektionsvorrichtung (10) eine kohärente Lichtquelle (11) aufweist.

4. Künstliche Augenlinse (1) nach Anspruch 3,
**dadurch gekennzeichnet, dass**
die künstliche Augenlinse (1) zumindest eine Haptik (5) aufweist, die mit dem optischen Teil (2) verbunden ist, wobei die kohärente Lichtquelle (11) an der Haptik (5) angeordnet ist.

5. Künstliche Augenlinse (1) nach einem der vorhergehenden Ansprüche,
**dadurch gekennzeichnet, dass**
die Bildprojektionsvorrichtung (10) ein holographisches Display (12) aufweist, das an dem optischen Teil (2) angeordnet ist.

6. Künstliche Augenlinse (1) nach Anspruch 4 und 5,
**dadurch gekennzeichnet, dass**
die künstliche Augenlinse (1) zumindest ein Lichtleiterelement (13) aufweist, mit welchem das Licht der kohärenten Lichtquelle (11) zum holographischen Display (12) geleitet ist.

7. Künstliche Augenlinse (1) nach einem der vorhergehenden Ansprüche,
**dadurch gekennzeichnet, dass**
an dem optischen Teil (2) eine Lochblende (14) der künstlichen Augenlinse (1) angeordnet ist.

8. Künstliche Augenlinse (1) nach Anspruch 7,
**dadurch gekennzeichnet, dass**
im Flächenbereich der Lochblende (14) hinter der Lochblende (14) zumindest eine Antenne (16) der künstlichen Augenlinse (1) und/oder zumindest ein holographisches Display (12) der künstlichen Augenlinse (1) angeordnet ist.

9. Künstliche Augenlinse (1) nach Anspruch 7 oder 8,
**dadurch gekennzeichnet, dass**
die Lochblende (14) zumindest bereichsweise als Solarzelle (15) ausgebildet ist und/oder die Lochblende (14) in dem optischen Teil (2) innen liegend angeordnet ist.

10. Künstliche Augenlinse (1) nach einem der vorhergehenden Ansprüche,
**dadurch gekennzeichnet, dass**
mit dem optischen Teil (2) Licht aus der Umgebung, das auf die künstliche Augenlinse (1) trifft, aufgrund der Ausgestaltung des optischen Teils (2) und der damit einhergehenden inhärenten optischen Abbildungseigenschaft dieses einfallende Umgebungslicht entsprechend umlenkbar ist, wobei die Bildprojektionsvorrichtung (10) hingegen nicht mit dem auf die künstliche Augenlinse (1) einfallenden Umgebungslicht arbeitet, sondern das Bild selbst erzeugt, und/oder die Bildprojektionsvorrichtung (10) zur Erzeugung von optischen Informationen unabhängig von dem auf die künstliche Augenlinse (1) aus der Umgebung einfallenden Umgebungslicht ausgebildet ist.

11. Elektronisches Netzhaut-Informationsdarstellungssystem, mit einer künstlichen Augenlinse (1) nach einem der vorhergehenden Ansprüche 1 bis 10, mit zumindest einem zur künstlichen Augenlinse (1) separaten und extern zur künstlichen Augenlinse (1) angeordneten Kommunikationsgerät (18) und mit einer drahtlosen Kommunikationsschnittstelle (19), mit welcher eine Übertragung von Informationssignalen zwischen der künstlichen Augenlinse (1) und dem Kommunikationsgerät (18) durchführbar ist.

## Claims

1. Artificial eye lens (1) having at least one optical part (2), which comprises a front side (3) and a back side (4) opposite the front side (3), wherein the artificial eye lens (1) comprises an image projection device (10),
**characterized in that**
the image projection device (10) is used to project optical information created by the image projection device (10) itself at least onto the back side (4) of the optical part (2), wherein the image projection device (10) enables image creation independently of the optical imaging property of the optical part (2), wherein the image projection device (10) is arranged on or in the optical part (2) such that it radiates the light image emitted thereby directly to the back side (4), and the image projection device (10) is designed to project optical information created by the image projection device (10) backwards through the back side (4) of the optical part (2) and out of the optical part (2).

2. Artificial eye lens (1) according to Claim **1,**
**characterized in that**
the image projection device (10) is designed to create a 3-D image.

3. Artificial eye lens (1) according to either of the preceding claims,
**characterized in that**
the image projection device (10) comprises a coherent light source (11).

4. Artificial eye lens (1) according to Claim **3,**
**characterized in that**
the artificial eye lens (1) comprises at least one haptic (5) that is connected to the optical part (2), wherein the coherent light source (11) is arranged on the haptic (5).

5. Artificial eye lens (1) according to any of the preceding claims,
**characterized in that**
the image projection device (10) comprises a holographic display (12) that is arranged on the optical part (2).

6. Artificial eye lens (1) according to Claims 4 and 5,
**characterized in that**
the artificial eye lens (1) comprises at least one light guide element (13), by means of which the light of the coherent light source (11) is guided to the holographic display (12).

7. Artificial eye lens (1) according to any of the preceding claims,
**characterized in that**
a perforated plate (14) of the artificial eye lens (1) is arranged on the optical part (2).

8. Artificial eye lens (1) according to Claim **7,**
**characterized in that**
at least one antenna (16) of the artificial eye lens (1) and/or at least one holographic display (12) of the artificial eye lens (1) is arranged in the surface region of the perforated plate (14) behind the perforated plate (14).

9. Artificial eye lens (1) according to Claim 7 or 8,
**characterized in that**
the perforated plate (14), at least in regions, takes the form of a solar cell (15) and/or
the perforated plate (14) is arranged in the interior of the optical part (2).

10. Artificial eye lens (1) according to any of the preceding claims,
**characterized in that**
the optical part (2) can be used to accordingly deflect light from the surroundings this incident ambient light, which is incident on the artificial eye lens (1), on account of the configuration of the optical part (2) and the associated inherent optical imaging property, wherein, by contrast, the image projection device (10) does not operate with the ambient light incident on the artificial eye lens (1) but rather creates the image itself, and/or the image projection device (10) is designed to create optical information independently of the ambient light incident on the artificial eye lens (1) from the surroundings.

11. Electronic retina information presentation system, comprising an artificial eye lens (1) according to any of the preceding Claims 1 to 10, comprising at least one communication apparatus (18) that is separate from the artificial eye lens (1) and arranged externally to the artificial eye lens (1), and comprising a wireless communication interface (19) by means of which information signals are transmittable between the artificial eye lens (1) and the communication apparatus (18).

## Revendications

1. Lentille intraoculaire artificielle (1), comprenant au moins une partie optique (2) qui présente une face avant (3) et une face arrière (4) opposée à la face avant (3), la lentille intraoculaire artificielle (1) présentant un dispositif de projection d'image (10),
**caractérisée en ce**
**qu'**une information optique produite par le dispositif de projection d'image (10) lui-même est projetée par le dispositif de projection d'image (10) au moins sur la face arrière (4) de la partie optique (2), dans laquelle le dispositif de projection d'image (10) permet une création d'image indépendamment de la propriété de reproduction optique de la partie optique (2), dans laquelle le dispositif de projection d'image (10) est disposé sur ou dans la partie optique (2) de telle sorte qu'il projette par rayonnement l'image lumineuse émise par celui-ci directement vers la face arrière (4), et le dispositif de projection d'image (10) est réalisé pour projeter une information optique produite par le dispositif de projection d'image (10) à travers la face arrière (4) de la partie optique (2) hors de la partie optique (2) vers l'arrière.

2. Lentille intraoculaire artificielle (1) selon la revendication 1,
**caractérisée en ce**
**que** le dispositif de projection d'image (10) est réalisé pour créer une image 3D.

3. Lentille intraoculaire artificielle (1) selon l'une quelconque des revendications précédentes,
**caractérisée en ce**
**que** le dispositif de projection d'image (10) présente une source de lumière (11) cohérente.

4. Lentille intraoculaire artificielle (1) selon la revendication 3,
**caractérisée en ce**
**que** la lentille intraoculaire artificielle (1) présente au moins une partie haptique (5) qui est reliée à la partie optique (2), la source de lumière (11) cohérente étant disposée au niveau de la partie haptique (5).

5. Lentille intraoculaire artificielle (1) selon l'une quelconque des revendications précédentes,
**caractérisée en ce**
**que** le dispositif de projection d'image (10) présente un dispositif d'affichage holographique (12) qui est disposé sur la partie optique (2).

6. Lentille intraoculaire artificielle (1) selon les revendications 4 et 5,
**caractérisée en ce**
**que** la lentille intraoculaire artificielle (1) présente au moins un élément de guide de lumière (13) qui permet de guider la lumière de la source de lumière (11) cohérente vers le dispositif d'affichage holographique (12).

7. Lentille intraoculaire artificielle (1) selon l'une quelconque des revendications précédentes,
**caractérisée en ce**
**qu'**un diaphragme à trou (14) de la lentille intraoculaire artificielle (1) est disposé sur la partie optique (2).

8. Lentille intraoculaire artificielle (1) selon la revendication 7,
**caractérisée en ce**
**que** dans la partie de surface du diaphragme à trou (14), derrière le diaphragme à trou (14), au moins une antenne (16) de la lentille intraoculaire artificielle (1) et/ou au moins un dispositif d'affichage holographique (12) de la lentille intraoculaire artificielle (1) sont disposés.

9. Lentille intraoculaire artificielle (1) selon la revendication 7 ou 8,
**caractérisée en ce**
**que** le diaphragme à trou (14) est réalisé au moins par endroits sous la forme d'une cellule solaire (15), et/ou le diaphragme à trou (14) est disposé en se trouvant à l'intérieur de la partie optique (2).

10. Lentille intraoculaire artificielle (1) selon l'une quelconque des revendications précédentes,
**caractérisée en ce**
**qu'**en raison de la configuration de la partie optique (2) et de la propriété de reproduction optique inhérente associée, la partie optique (2) permet de dévier de manière adéquate la lumière provenant de l'environnement cette lumière environnante incidente qui est incidente sur la lentille intraoculaire artificielle (1), dans laquelle le dispositif de projection d'image (10) par contre ne travaille pas avec la lumière environnante incidente sur la lentille intraoculaire artificielle (1) mais crée lui-même l'image, et/ou le dispositif de projection d'image (10) est réalisé pour produire des informations optiques indépendamment de la lumière environnante incidente sur la lentille intraoculaire artificielle (1) à partir de l'environnement.

11. Système électronique de représentation d'informations de rétine, comprenant une lentille intraoculaire artificielle (1) selon l'une quelconque des revendications 1 à 10, comprenant au moins un appareil de communication (18) séparé de la lentille intraoculaire artificielle (1) et disposé à l'extérieur de la lentille intraoculaire artificielle (1), et une interface de communication sans fil (19) qui permet d'effectuer une transmission de signaux d'information entre la lentille intraoculaire artificielle (1) et l'appareil de communication (18).
